# EUROPÄISCHE PATENTANMELDUNG

(11) **EP 1 526 122 A2**
(43) Veröffentlichungstag der Anmeldung: **27.04.2005**
(21) Anmeldenummer: 04024475.8
(22) Anmeldetag: 14.10.2004
(51) Int. Cl.: C07C 37/00, C07C 41/18, C07C 209/72, C07C 39/14, C07C 39/17, C07C 43/21, C07C 211/60

(54) **Verfahren zur Reduktion von Binaphtylderivaten**

(30) Priorität: 21.10.2003 DE 10349399
(71) Anmelder: Degussa AG, 40474 Düsseldorf (DE)
(72) Erfinder: Börner, Armin Prof.Dr., 18055 Rostock (DE); Korostylev, Andrei Dr., 390006 Ryazan (RU); Tararov, Vitali Dr., 113525 Moskau (RU); Monsees, Axel Dr., 60487 Frankfurt (DE)

(57) **Zusammenfassung**

Die Erfindung betrifft ein Verfahren zur Herstellung von H₈-1,1'-bi-2-naphthylderivaten der Formel (II) 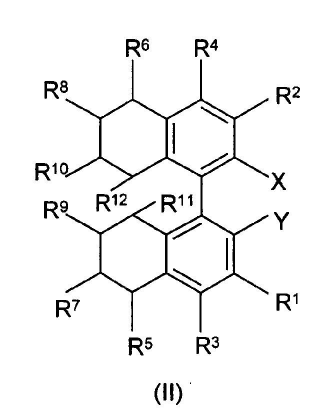 durch Reduktion der entsprechenden Binaphthylderivate mit Wasserstoff in Gegenwart eines Katalysators enthaltend mindestens ein Metall ausgewählt aus der Gruppe Pt, Ir, Os, Pd, Rh, Ru, Ni, Co und Fe aufgebracht auf einem festen Träger.

## Beschreibung

Die Erfindung betrifft ein neues Verfahren zur selektiven Reduktion von Binaphthylderivaten mit Wasserstoff.

2,2'-funktionalisierte 1,1'-bi-2-Naphthylderivate, wie z. B. BINOL (1,1'-bi-2-Naphtol) oder BINAM (1,1'-bi-2-Naphtylamin) sind bewährte chirale Liganden in der asymmetrischen Katalyse. Insbesondere davon abgeleitete Liganden, die auf einem H₈-Binaphthylgrundgerüst basieren sind in das Blickfeld der weiteren Entwicklung gelangt, da diese Liganden im Vergleich oft eine höhere asymmetrische Induktion zeigen. Folglich sind z. B. enantiomerenreine H₈-1,1'-bi-2-Naphthole oder H₈-1,1'-bi-2-Naphthylamine selbst sehr begehrte Liganden bzw. begehrte Ausgangsverbindungen zu ihrer Derivatisierung.

H₈-1,1'-bi-2-Naphthylderivate sind durch Reduktion der entsprechenden 2,2'funktionalisierte 1,1'-bi-2-Naphthylderivate erhältlich. In der Regel wird dazu ein von Cram et al. (Angew. Chem. 2001, 113, Nr.8, 1500-1504) entwickeltes Verfahren, bei dem die Reduktion mit Hilfe eines PtO₂-Katalysators in Gegenwart von Essigsäure erfolgt, zurückgegeriffen. Zur Durchführung des Verfahrens werden relativ große Katalysatormengen benötigt. Weiterhin kann die Reaktion nur bei geringen Temperaturen enantioselektiv durchgeführt werden, woraus sich lange Reduktionszeiträume ergeben.

Ein weiteres Reduktionsverfahren wird in Hui Guo, et al. Tetrahedron Letters 41, (2000), 10061-10064 beschrieben, wobei als Katalysator eine Raney Ni/Al Verbindung in einer wässrig-alkalischen Isopropanollösung verwendet wird. Bei dem dort beschriebenen Verfahren sind ebenfalls lange Reaktionszeiten, um relativ geringen Ausbeuten zu erzielen, nötig.

Der vorliegenden Erfindung lag die Aufgabe zugrunde ein Verfahren zur Herstellung von H₈-1,1'-bi-2-naphthylderivaten mit hoher Selektivität bereitzustellen, wobei die gewünschten Produkte mit guten Ausbeuten erhalten werden können.

Die Aufgabe wird durch eine katalytische Reduktion von Binaphthylen mit Wasserstoff unter Verwendung eines Katalysators enthaltend mindestens ein, auf einen festen Träger aufgebrachtes Metall aus der achten Nebengruppe, gelöst.

Folglich ist Gegenstand der vorliegenden Erfindung ein Verfahren
zur Reduktion von Binaphthylderivaten der Formel (I), 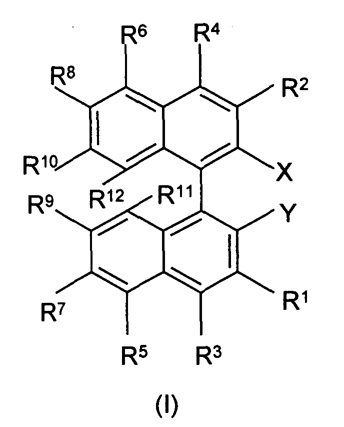 zu H₈-1,1'-bi-2-naphthylderivaten (5,5',6,6',7,7',8,8'-octahydro-1,1'-dinaphthylderivate) der Formel (II) 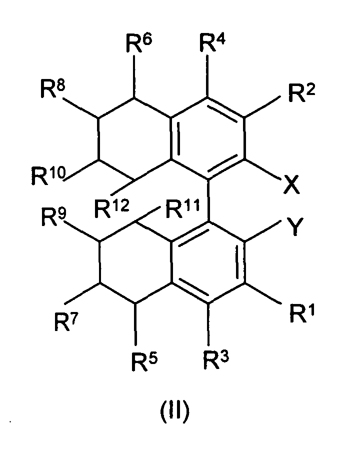 mit Wasserstoff
in Gegenwart eines Katalysators enthaltend mindestens ein Metall ausgewählt aus der Gruppe Pt, Ir, Os, Pd, Rh, Ru, Ni, Co und Fe, wobei das oder die Metalle auf einem festen Träger aufgebracht sind,
wobei
- X und Y: unabhängig voneinander für einen Rest ausgewählt aus der Gruppe OH, OR', O-((CₙH₂ₙ)-O-)ₘR', NH₂, NHR', NR'R", SH und SR', worin R' und R" unabhängig voneinander für einen Wasserstoff-, (C₁-C₂₄)-Alkyl-, (C₃-C₁₂)-Cycloalkyl-, (C₂-C₂₄)-Alkenyl-, (C₃-C₂₄)-Cycloalkenyl- oder (C₅-C₂₀)-Aryl- Rest stehen,
wobei die Reste R' und R" auch weitere Substituenten tragen können und worin n für eine ganze Zahl von 1 bis 24, bevorzugt von 1 bis 6 und m für eine ganze Zahl von 1 bis 12, bevorzugt von 1 bis 3 steht,
und worin die Reste
R¹, R², R³, R⁴, R⁵, R⁶, R⁷, R⁸, R⁹, R¹⁰, R¹¹ und R¹² unabhängig voneinander für einen Rest ausgewählt aus der Gruppe Wasserstoff, (C₁-C₂₄)-Alkyl, (C₃-C₁₂)-Cycloalkyl, (C₂-C₂₄)-Alkenyl, (C₃-C₂₄)-Cycloalkenyl, (C₅-C₂₀)-Aryl, Si((C₁-C₂₄)-Alkyl)₃, Si(C₁-C₂₄)-Alkyl)₂(C₆-Aryl), Si(C₁-C₂₄)-Alkyl)(C₆-Aryl)₂, Si(C₆-Aryl)₃, S((C₁-C₂₄)-Alkyl), S((C₅-C₂₀)-Aryl), F, Cl, Br oder I stehen,
wobei die Reste R² und R⁴ und R¹ und R³ unabhängig voneinander auch miteinander verknüpft sein können und
wobei die genannten Reste R¹ bis R¹² auch weitere Substituenten tragen können.

Als Katalysator haben sich insbesondere Metalle der achten Nebengruppe bewährt, die auf einen porösen Träger, die z. B. in Pulver oder Granulatform vorliegen können und bevorzugt eine spezifische Oberfläche zwischen 0,1 m²/g und 5000 m²/g, bevorzugt zwischen 1 m²/g und 2500 m²/g besitzen können, aufgebracht wurden. Besonders bevorzugt werden Katalysatoren enthaltend Palladium, Rhodium und/oder Ruthenium bei der Durchführung der Reduktion. Bevorzugte poröse Träger sind insbesondere Kohlenstoffträger, wie z. B. Aktivkohle, oder Aluminiumoxidträger, wie z. B. Al₂O₃, wobei die Aktivkohleträger ein bevorzugtes Schüttgewicht zwischen 100 und 450 g/l, besonders bevorzugt zwischen 200 und 400 g/l und/oder eine spezifische Oberfläche zwischen 700 und 2000 m²/g, besonders bevorzugt zwischen 900 und 1800 m²/g und die Al₂O₃ -Träger ein bevorzugtes Schüttgewicht zwischen 200 und 800 g/l, besonders bevorzugt zwischen 250 und 750 g/l und/oder eine spezifische Oberfläche zwischen 2 und 300 m²/g, besonders bevorzugt zwischen 5 und 250 m²/g besitzen. Besonders bevorzugte Katalysatoren sind dementsprechend Pd/C, Ru/C, Pd/Al₂O₃ oder Ru/Al₂O₃ deren Träger den eben beschriebenen Spezifikationen entsprechen.

Darüber hinaus ist es ausreichend, den Katalysator in relativ geringen Mengen zu verwenden. Bevorzugt wird der Katalysator in einer Menge eingesetzt, bei der das Metall in einem Bereich von 0,01 mol % bis 15 mol %, besonders bevorzugt in einer Menge von 0,1 mol % bis 10 mol %, insbesondere zwischen 0,5 mol % und 5 mol % gegenüber der Substratmenge vorliegt.

Mit dem bereitgestellten Verfahren werden die gewünschten Produkte der Formel (II) in hoher Ausbeute erhalten, wobei die hohen Ausbeuten auch bei relativ milden Reduktionsbedingungen, wie z. B. bei Temperaturen von unter 120 °C und einem anfänglichen Wasserstoffdruck von unter 80 bar. Dies scheint auch der Grund für die sehr gute Selektivität der Umsetzung zu den H₈-1,1'-bi-2-naphthylderivaten der Formel (II) zu sein, eine Racemisierung der Produkte während der Herstellung kann weitgehend oder sogar ganz vermieden werden. Ein weiterer Vorteil des beschriebenen Verfahrens besteht in der guten Recyclebarkeit des Katalysators. So konnten z. B. die in den Ausführungsbeispielen eingesetzten Katalysatoren mehrfach wiedergewonnen und ohne feststellbaren Aktivitätsverlust erneut eingesetzt werden. Folglich ist das beschriebenen Verfahren insbesondere für die technische Herstellung von Verbindungen der Formel (II) von Interesse. Die Wiedergewinnbarkeit des Katalysators ohne Aktivitätsverlust könnte an den Reaktionsbedingungen, wie z. B. der Metall/Träger-Konstruktion, den milden Reaktionsbedingungen oder dem Wegfall katalysatorschädigender Reaktionszusätze, wie z. B. Säuren, liegen. Durch die saubere Reaktionsführung ist auch die Aufarbeitung der erhaltenen Produkte sehr einfach.

Bevorzugt wird das beanspruchte Reduktionsverfahren bei Temperaturen von 30 °C bis 100 °C, besonders bevorzugt von 40 °C bis 70 °C durchgeführt. Der anfänglich angelegt Wasserstoffdruck liegt bevorzugt zwischen 1 bar und 70 bar, besonders bevorzugt zwischen 10 bar und 50 bar.

Bevorzugte Ausgangsstoffe für die beanspruchte Reduktion sind Binaphthylderivate der Formel (I) deren Reste X und Y unabhängig voneinander ausgewählt sind aus der Gruppe OH, OR', OCH₂OCH₃, OCH₂OCH₂CH₃, OCH₂OCH₂OCH₃, SH, SR', NH₂, NHR' und NR'R", wobei R' und R" unabhängig voneinander für einen (C₁-C₁₂)-Alkyloder C₆-Arylrest stehen. Bevorzugte Reste R¹ bis R¹² sind unabhängig voneinander ausgewählt ist aus der Gruppe Wasserstoff, (C₁-C₁₂)-Alkyl, (C₃-C₆)-Cycloalkyl und C₆-Aryl. Bevorzugte sind die Reste R¹, R², R⁷ und R⁸ von Wasserstoff abweichende Substituenten. Besonders bevorzugt Ausgangsverbindungen sind Binaphthylderivate der Formel (I) deren Reste R¹ bis R¹² Wasserstoff sind.

Die einzelnen Reste R', R" und R¹ bis R¹² können jeweils unabhängig voneinander weitere Substituenten tragen, die bevorzugt aus der Gruppe H, OH, OR', O-((CₙH₂ₙ)-O-)ₘR''', NH₂, NHR''', NR'''R'''', SH, SR''', (C₁-C₂₄)-Alkyl, (C₃-C₁₂)-Cycloalkyl, (C₂-C₂₄)-Alkenyl, (C₃-C₂₄)-Cycloalkenyl, (C₅-C₂₀)-Aryl, Si((C₁-C₂₄)-Alkyl)₃, Si(C₁-C₂₄)-Alkyl)₂(C₆-Aryl), Si(C₁-C₂₄)-Alkyl)(C₆-Aryl)₂, Si(C₆-Aryl)₃, S((C₁-C₂₄)-Alkyl), S((C₅-C₂₀)-Aryl), F, Cl, Br oder I stehen, ausgewählt sind, wobei die Reste R''' und R"" die für R' und R" genannte Bedeutung haben und die Indizes n und m die oben genannte Bedeutung besitzen.

Da aromatische Substituenten während der Reduktion in der Regel ebenfalls hydriert werden, können Edukte mit ungesättigten Substituenten bzw. mit Arylsubstituenten zur Herstellung der entsprechenden Alkyl- oder Cycloalkylverbindungen genutzt werden. Umgekehrt kann es nötig sein reduktionsanfällige ungesättigte oder aromatische Substituenten erst nach der Reduktion einzuführen. Insbesondere können, unabhängig voneinander, die Reste R', R", R¹, R², R⁵, R⁶, R⁷, R⁸, R⁹, R¹⁰, R¹¹ und R¹² nach erfolgter Reduktion eingeführt werden. Dazu stehen eine Reihe bekannter Derivatisierungsverfahren, wie z. B. in Cox, J. P., et al. , Tetrahedron Letters, Vol. 33, No. 17, 2253-2256, 1992; Simonsen, K. B., et al., J. Org. Chem., 1998, 63, 7536-7538 oder in WO 02/40491 beschrieben, bereit.

Mit dem erfindungsgemäßen Verfahren können enantioselektiv angereicherte Produkte der Formel (II) erhalten werden, bevorzugt werden die gewünschten Produkte mit einer Enantioselektivität (ee) von über 80 %, besonders bevorzugt von über 90 %, insbesondere von über 95 % erreicht. In vielen Fällen können sogar praktisch enantiomerenreine Verbindungen erhalten werden, die eine Enantiomerenreinheit von über 99 % aufweisen.

Die mit dem beanspruchten Verfahren erhaltenen Verbindungen der Formel (II) können z. B. als Liganden in katalytischen asymmetrischen Reaktionen oder Polymerisationen, insbesondere bei der asymmetrischen Hydrierung, der asymmetrischen Alkylierung von Aldehyden oder in hetero-Diels-Alder-Reaktionen eingesetzt werden. Darüber hinaus können Sie als Ausgangsstoff für weitere Derivatisierungen dienen, um optimierte Liganden für solche asymmetrische Katalysereaktionen bereitzustellen.

### Ausführungsbeispiele:

### Allgemeines:

Alle verwendeten Ausgangsverbindungen sind, sofern nicht anders erwähnt, kommerziell erhältlich und wurden ohne weitere Reinigung eingesetzt.
Die zur Charakterisierung der Produkte aufgenommenen NMR-Spektren wurden bei 400.13 (¹H) MHz und 100.63 (¹³C) MHz mit TMS als Standrad aufgenommen. Die angegebenen Werte J sind Hz, δ in ppm angegeben.
Die chirale HPLC wird mit einer Chiralcel Säule, Hexan/EtOH = 90/10 (Octahydrobinaphthole) bzw. einer Chiralcel Säule, Hexan/EtOH = 99.5/0.5 (3,3'dimethyl-octahydrobinaphthole) oder einer (R,R)-Whelk-01 Säule; Hexan/EtOH = 99.95/0.05 (3,3'-dicyclohexyl-octahydrobinaphthole) durchgeführt.

### Beispiele 1 bis 12: Hydrierung von Binaphtholen

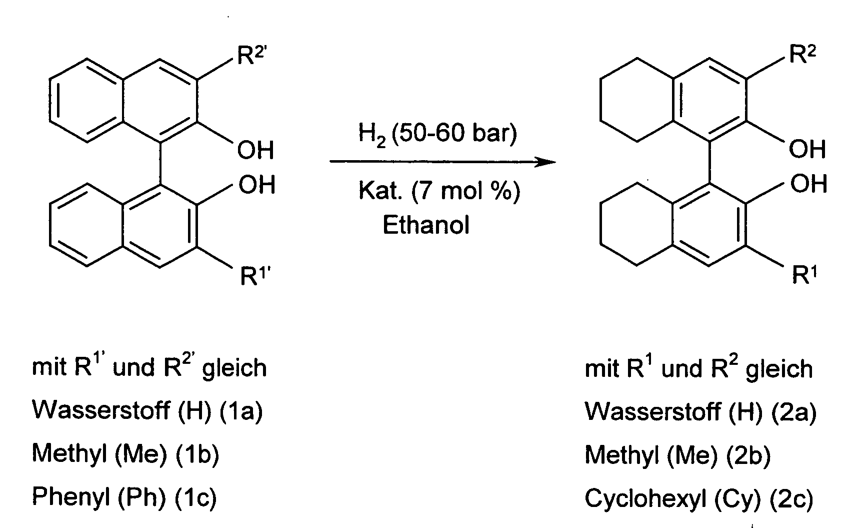

### a) Synthese der Ausgangsstoffe (1 b) und (1 c):

Ausgehend von BINOL wurden die Verbindungen 1 b und 1 c gemäß der Vorschrift von Arnold, L.A., et al., Tetrahedron Letters, 2000, 56, 2865-2878 hergestellt.

### b) Reduktion der Substrates 1c:

0.438 g (1 mmol) des Substrates werden mit 0.3 g 5 %igem Pd/C (50 % nass) und 10 ml Ethanol in einen 35 ml Autoklaven gegeben und unter 50 bar Wasserstoffdruck bei 70 °C 7 h gerührt. Nach 7 h war kein Wasserstoffverbrauch mehr feststellbar. Das Reaktionsgemisch wurde auf Raumtemperatur abgekühlt. Der Katalysator wurde anschließend abfiltriert und mehrmals mit CH₂Cl₂ (3 X 10 ml) gewaschen. Die erhaltenen Filtrate wurden kombiniert und im Vakuum eingeengt. Analytisch reines Reduktionsprodukt konnte mittels Flashchromatographie (Silica, CH₂Cl₂) erhalten werden. Die NMR-Analyse des Produktes 2c zeigt an, dass keine Produkt oder Produkt/Edukt-Mischung vorliegt. Ausbeute 100 %, > 99 % ee

Analytische Daten zu (+)-(*R*)-3,3'-Dicyclohexyl-5,5',6,6',7,7',8,8'-octahydro-2,2'dihydroxy-1,1'-dinaphthyl (2c):
mp 118-120°C;
[α]_{D}²⁵ +41.7 (c=1, CH₂Cl₂); EI-MS *m*/*z* 458 (M⁺, 100);
¹H NMR (400 MHz, CDCl₃)
δ6.89 (s, 2H), 4.55 (s, 2H), 2.80 (m, 2H), 2.65 (m, 4H), 2.00-2.19 (m, 4H), 1.71-1.89 (m, 8H), 1.53-1.70 (m, 10H), 1.34 (m, 8H), 1.18 (m, 2H);
¹³C NMR (100 MHz, CDCl₃)
δ149.0, 134.3, 132.1, 129.9 (all C), 128.5 (CH), 119.2 (C), 37.7 (CH), 33.6, 29.8, 27.5, 27.3, 26.9, 23.6 (all CH₂). Anal. Calcd. for C₃₂H₄₂O₂: C, 83.79; H, 9.23. Found: C, 83.08; H, 9.62.

Analog zu b) wurden weitere Reduktionen mit unterschiedlichen Substraten und Katalysatoren durchgeführt, die Ergebnisse sind in Tabelle 1 zusammengefasst.

**Tabelle 1:**

| Ergebnisse der katalytischen Hydrogenierung der Substrate 1 a, 1 b, 1 c. | | | | | | |
|---|---|---|---|---|---|---|
| Bsp. Nr. | Substrat | Katalysator | T (in °C) | Reaktionszeit (in Stunden) | Ausbeute (in %) | ee (in %) |
| 1 | 1a | Ru/Al₂O₃ | 100 | 3 | 100 | 97.2 |
| 2 | 1a | Ru/C | 50 | 1.5 | 98 | >99 |
| 3 | 1a | Ru/C | 70 | 0.5 | 98 | >99 |
| 4 | 1a | Pd/C | 50 | 2.5 | 99 | >99 |
| 5 | 1a | Pd/C | 70 | 0.5 | 99 | >99 |
| 6 | 1b | Ru/Al₂O₃ | 100 | 2 | 100 | 99.0 |
| 7 | 1b | Ru/C | 50 | 7 | 99 | >99 |
| 8 | 1b | Ru/C | 70 | 0.5 | 97 | >99 |
| 9 | 1b | Pd/C | 70 | 0.5 | 98 | >99 |
| 10 | 1c | Ru/C | 70 | 7 | 96 | 98.5 |
| 11 | 1c | Pd/C | 70 | 7 | 100 | >99 |
| 12 | 1c | Pd/C | 100 | 0.75 | 100 | 99.0 |

Es zeigt sich, dass bei der Reduktion von BINOL und BINOL-Derivaten die verwendeten Katalysatoren die gewünschten Produkte in hoher Enantioselektivität bei hohen Ausbeuten erhältlich sind. Pd und Ru -haltige Katalysatoren auf Basis eines Kohlenstoffträgers erlauben dabei eine Umsetzung bei niedriger Temperatur, aber auch die Umsetzung mit Katalysatoren auf Aluminiumoxidbasis führt zu sehr guten Ergebnissen, wobei keine Racemisierung auch bei 100 °C Reaktionstemperatur feststellbar ist. Keine nennenswerten Unterschiede sind bezüglich der katalytischen Aktivität des eingesetzten Palladiums und Rutheniums feststellbar.

Mit den Pd/C oder Ru/C-Katalysatoren sind schon nach 30 Minuten bei einer Temperatur von 70 °C nahezu quantitative Ausbeuten bei der Umsetzung von 1a und 1 b erzielbar. Längere Reaktionszeiten sind bei der Reduktion von 1 c nötig, was an dem zusätzlichen Phenylresten liegen dürfte, die ebenfalls unter den gegebenen Bedingungen reduziert werden.

Die Untersuchung der Produkte mit Hilfe der Chiral-HPLC wurde festgestellt, dass die erhaltenen Produkte keiner nachweisbaren Racemisierung während der Reduktion unterlagen, die Produkte 2a, 2b, 2c wurden mit einer Enantioselektivität von über ≥99% ee erhalten.

### Beispiele 13 bis 19: Hydrierung von Dialkoxybinaphthylderivaten

3,3'-disubstitutierte chirale H₈-BINOLe 2a, 2b, 2c können ebenfalls durch Hydrierung der entsprechenden bis-methylierten Verbindungen 3a, 3b, 3c mit anschließender Demethylierung erhalten werden. Die Hydrierung der 2,2-Dimethoxybinaphthylderivate 3a, 3b, 3c erfolgt gemäß der Vorschrift unter Beispiel 1 b). Im folgenden werden dann die bei der Reduktion erhaltenen 2,2'-dimethoxy-1,1'binaphthylderivate 4a, 4b, 4c unter Zugabe von BBr₃ entschützt.

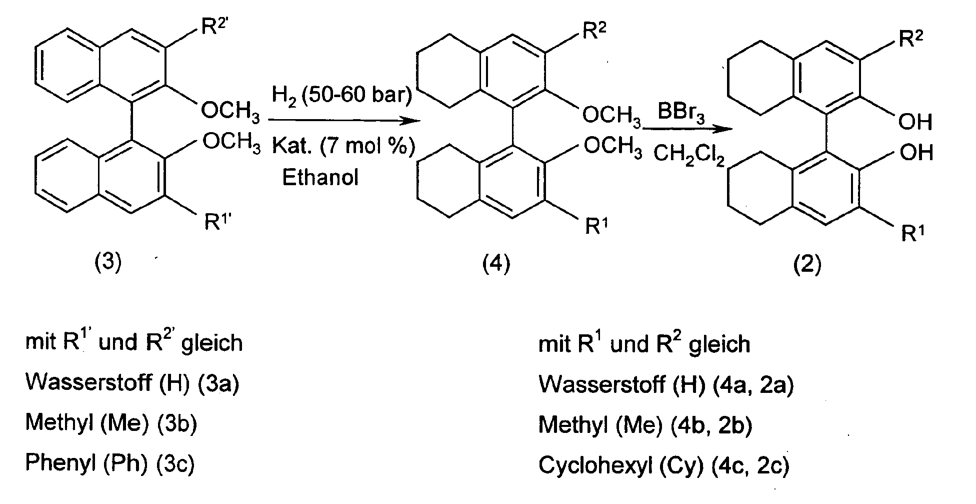

Die Hydrierung verläuft mit quantitativen Ausbeuten, allerdings bei etwas längeren Reaktionszeiten gegenüber Beispiel 1. Die nach der Entschützung erhaltenen Produkte 2a, 2b und 2c liegen in geringem Masse racemisiert vor, die Produkt enthalten 1-5 % des durch Racemisierung entstandenen Enantiomers. Die Ergebnisse der Umsetzungen sind in Tabelle 2 wiedergegeben.

**Tabelle 2.**

| Katalytische Reduktion der 2,2'-Dimethoxy-binaphthylen 2a, 2b, 2c. | | | | | | |
|---|---|---|---|---|---|---|
| Bsp. Nr. | Substrat | Katalysator | T (in °C) | Reaktionszeit (in Stunden) | Ausbeute^{a} (in %) | ee^{b} (in %) |
| 13 | 3a | Ru/C | 100 | 0.5 | 74 | 95.4 |
| 14 | 3a | Pd/C | 50 | 3 | 80 | 97.0 |
| 15 | 3a | Pd/C | 70 | 1.5 | 77 | 97.2 |
| 16 | 3b | Ru/C | 100 | 1.5 | 71 | 93.3 |
| 17 | 3b | Pd/C | 100 | 1 | 75 | 90.2 |
| 18 | 3c | Ru/C | 100 | 0.5 | 69 | 98.8 |
| 19 | 3c | Pd/C | 100 | 1 | 74 | 98.5 |

| | | | | | | |
|---|---|---|---|---|---|---|
| ^{a}Ausbeute an entschützten Binaphthol 2a, 2b, 2c. | | | | | | |
| ^{b} Bestimmt durch chirale HPLC der entschützten Binaphthole 2a, 2b, 2c. | | | | | | |

### Beispiel 20: Reduktion von Diaminobinaphthylderivaten

Die Hydrierung von (R)-2,2'-diamino-1,1'-binaphthylderivaten der Formel 5a in die entsprechenden H₈-Derivative 6a erfolgt bei 100°C mit einem Pd/C-Katalysator (7 mol % Pd bezogen auf das Substrat) in 30 min.

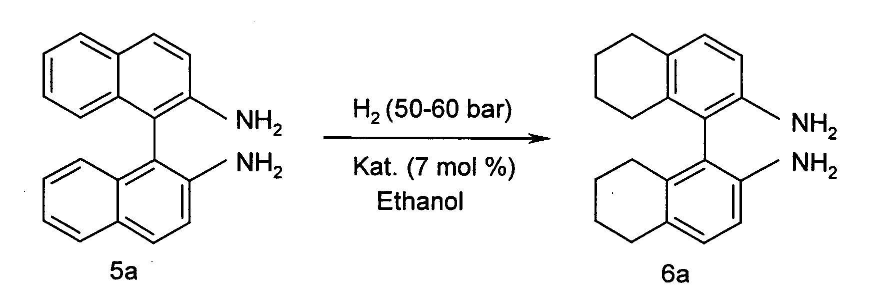

Die Umsetzung verläuft quantitative (Ausbeute 97% bezogen auf das Edukt), es konnte mittels Chiral-HPLC keine Racemisierung der erhaltenen Produkte festgestellt werden (>99% ee).

### Beispiel 21: Herstellung von (R)-H⁸-BINOL 2a durch Reduktion der Verbindung 1a im präparativen Maßstab

Um die technische Eignung des Verfahrens zu prüfen wurde ein größerer Ansatz von 20 g der Verbindung 1 a bei 100°C und einem Anfangswasserstoffdruck von 80 bar in Gegenwart eines Pd/C -Katalysators wie folgt umgesetzt:

20.0 g (70 mmol) der Verbindung 1a, 2.97 g 5%igem Pd/C (50% nass) und 100 ml Ethanol wurden in einem 300ml Autoklaven gegeben und unter 80 bar Wasserstoffdruck bei 100°C 5.5 h gerührt. Im Anschluss wurde das Reaktionsgemisch auf Raumtemperatur abgekühlt, der Katalysator abfiltriert und mehrmals mit Ethanol (3x50 ml) gewaschen. Die erhaltenen Filtrate wurden kombiniert und im Vakuum eingeengt. Es wurden 20.1 g (98% Ausbeute) von spektrometrisch reinem (*R*)-H⁸-BINOL 2a (weißer Feststoff / 99.7% ee) erhalten.

### Beispiel 21: Katalysatorrecycling

Zur Bestimmung der Recyclebarkeit des Katalysators wird die folgende Reaktion mehrfach durchgeführt

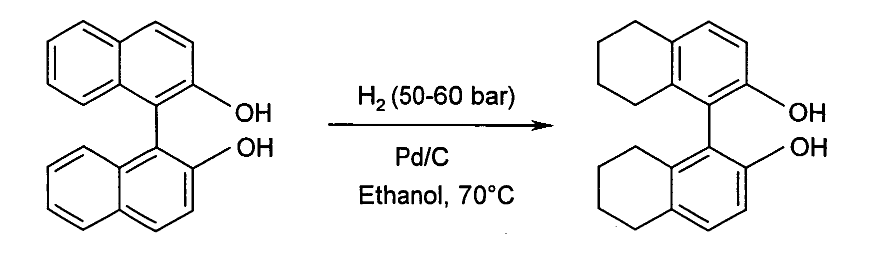

Als Katalysator wird ein 5%iger Palladiumkatalysator auf Aktivkohle (Degussa AG, Typ E 10 R) eingesetzt. Die Umsetzung und Wiedergewinnung des Katalysators erfolgt analog der Vorschrift aus Beispiel 1 b). In der folgenden Tabelle sind die Ergebnisse der Umsetzung zusammengefasst.

| Umsetzung | Reaktionszeit | Ausbeute (%) | ee (%) |
|---|---|---|---|
| 1 | 60 | 99 | >99 |
| 2 | 80 | 98 | >99 |
| 3 | 70 | 100 | >99 |
| 4 | 60 | 98 | >99 |
| 5 | 60 | 99 | >99 |

Selbst nach 5 Umsetzungen (4 Recyclingcylen) ist kein Aktivitätsverlust bezüglich des Katalysators feststellbar.

## Patentansprüche

1. Verfahren zur Reduktion von Binaphthylderivaten der Formel (I), 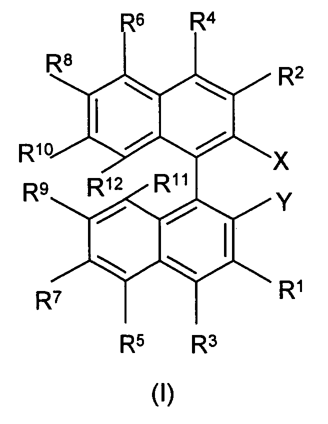 zu H₈-1,1'-bi-2-naphthylderivaten der Formel (II) 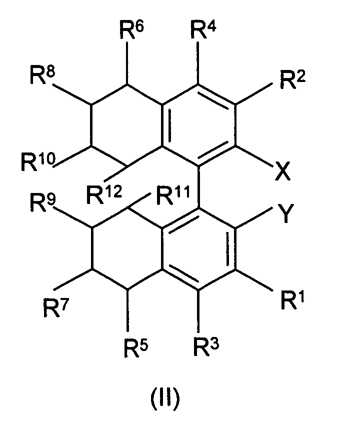 mit Wasserstoff
in Gegenwart eines Katalysators enthaltend mindestens ein Metall ausgewählt aus der Gruppe Pt, Ir, Os, Pd, Rh, Ru, Ni, Co und Fe, wobei das oder die Metalle auf einem festen Träger aufgebracht sind,
wobei
X und Y unabhängig voneinander für einen Rest ausgewählt aus der Gruppe OH, OR', O-((CₙH₂ₙ)-O-)ₘR', NH₂, NHR', NR'R", SH und SR', worin R' und R" unabhängig voneinander für einen Wasserstoff-, (C₁-C₂₄)-Alkyl, (C₃-C₁₂)-Cycloalkyl, (C₂-C₂₄)-Alkenyl, (C₃-C₂₄)-Cycloalkenyl oder (C₅-C₂₀)-Aryl -Rest stehen,
wobei die Reste R' und R" auch weitere Substituenten tragen können und worin n für eine ganze Zahl von 1 bis 24, bevorzugt von 1 bis 6 und m für eine ganze Zahl von 1 bis 12, bevorzugt von 1 bis 3 steht,
und worin die Reste
R¹, R², R³, R⁴, R⁵, R⁶, R⁷, R⁸, R⁹, R¹⁰, R¹¹ und R¹² unabhängig voneinander für einen Rest ausgewählt aus der Gruppe Wasserstoff, (C₁-C₂₄)-Alkyl, (C₃-C₁₂)-Cycloalkyl, (C₂-C₂₄)-Alkenyl, (C₃-C₂₄)-Cycloalkenyl, (C₅-C₂₀)-Aryl Si((C₁-C₂₄)-Alkyl)₃, Si(C₁-C₂₄)-Alkyl)₂(C₆-Aryl), Si(C₁-C₂₄)-Alkyl)(C₆-Aryl)₂, Si(C₆-Aryl)₃, S((C₅-C₂₄)-Alkyl), S((C₅-C₁₀)-Aryl), F, Cl, Br oder I stehen,
wobei die Reste R² und R⁴ und R¹ und R³ unabhängig voneinander auch miteinander verknüpft sein können und
wobei die genannten Reste R¹ bis R¹² auch weitere Substituenten tragen können.

2. Verfahren nach Anspruch 1, **dadurch gekennzeichnet, dass** das oder die Metalle auf einem porösen Träger mit einer spezifischen Oberfläche zwischen 0,1 m²/g und 5000 m²/g aufgebracht sind.

3. Verfahren nach Anspruch 1 oder 2, **dadurch gekennzeichnet, dass** das oder die Metalle auf einem Aktivkohle- oder Aluminiumoxid-Träger aufgebracht sind.

4. Verfahren nach einem der Ansprüche 1 bis 3, **dadurch gekennzeichnet, dass** die Reduktion bei einer Temperatur von unter 120 °C durchgeführt wird.

5. Verfahren nach Anspruch 4, **dadurch gekennzeichnet, dass** die Reduktion bei einer Temperatur von 30 °C bis 100 °C durchgeführt wird.

6. Verfahren nach einem der Ansprüche 1 bis 5, **dadurch gekennzeichnet, dass** die Reduktion bei einem anfänglichen Wasserstoffdruck von unter 80 bar durchgeführt wird.

7. Verfahren nach Anspruch 6, **dadurch gekennzeichnet, dass** die Reduktion bei einem anfänglichen Wasserstoffdruck von unter 60 bar durchgeführt wird.

8. Verfahren nach einem der Ansprüche 1 bis 7, **dadurch gekennzeichnet, dass** als Edukt Binaphthylderivate der Formel (I) eingesetzt werden deren Reste X und Y unabhängig voneinander ausgewählt sind aus der Gruppe OH, OR', OCH₂OCH₃, OCH₂OCH₂CH₃, OCH₂OCH₂OCH₃, SH, SR', NH₂, NHR' und NR'R", wobei R' und R" unabhängig voneinander für einen (C₁-C₁₂)-Alkyl-, (C₃-C₆)-Cycloalkyl- oder C₆-Arylrest stehen.

9. Verfahren nach einem der Ansprüche 1 bis 8, **dadurch gekennzeichnet, dass** als Edukt Binaphthylderivate der Formel (I) eingesetzt werden worin mindestens einer der Reste R¹ bis R¹² unabhängig voneinander ausgewählt ist aus der Gruppe Wasserstoff, (C₁-C₁₂)-Alkyl, (C₃-C₆)-Cycloalkyl und C₆-Aryl.

10. Verfahren nach Anspruch 9, **dadurch gekennzeichnet, dass** als Edukt Binaphthylderivate der Formel (I) eingesetzt werden deren Reste R¹ bis R¹² Wasserstoff sind.

11. Verwendung von Katalysatoren enthaltend mindestens ein Metall aus der Gruppe Pt, Ir, Os, Pd, Rh, Ru, Ni, Co und Fe, wobei das oder die Metalle auf einem festen Träger aufgebracht sind zur Reduktion von Binaphthylderivaten mit Wasserstoff.

12. Verwendung von Katalysatoren gemäß Anspruch 11 enthaltend mindestens ein Metall aus der Gruppe Pd, Rh und Ru, wobei das oder die Metalle auf einem festen Träger aufgebracht sind zur Reduktion von Binaphthylderivaten mit Wasserstoff.

13. Verwendung von Katalysatoren gemäß Anspruch 11 oder 12 enthaltend als festen Träger einen Aktivkohleträger oder einen Aluminiumoxidträger.
